# EUROPÄISCHE PATENTANMELDUNG

(11) **EP 1 983 061 A1**
(43) Veröffentlichungstag der Anmeldung: **22.10.2008**
(21) Anmeldenummer: 08007079.0
(22) Anmeldetag: 10.04.2008
(51) Int. Cl.: C12Q 1/56, G01N 33/557, G01N 33/86

(54) **Verfahren zur Bestimmung der Reaktions-Lag-Phase in einer analytabhängigen Reaktion**

(30) Priorität: 17.04.2007 DE 102007017906
(71) Anmelder: Siemens Healthcare Diagnostics Products GmbH, 35041 Marburg (DE)
(72) Erfinder: Weyl, Andreas, Dr., 35117 Münchhausen (DE); Henckel, Thilo, Dr., 6017 Ruswil (CH)

(57) **Zusammenfassung**

Die Erfindung liegt auf dem Gebiet der Analysetechnik und betrifft ein verbessertes Verfahren zur Bestimmung der Konzentration oder Aktivität eines Analyten, wobei eine analytabhängige Nachweisreaktion in Gang gesetzt wird und die ermittelte Reaktionskinetik ausgewertet wird. Das Verfahren ermöglicht für jede Messung eine individuelle Bestimmung der Lag-Phase der Reaktionskinetik.

## Beschreibung

Die Erfindung liegt auf dem Gebiet der Analysetechnik und betrifft ein verbessertes Verfahren zur Bestimmung der Konzentration oder Aktivität eines Analyten in einer Probe.

In der modernen Analysetechnik kommt je nach Art des zu bestimmenden Analyten eine Vielzahl von unterschiedlichen Verfahrensprinzipien zum Einsatz. Um zum Beispiel Proteine oder Peptide quantitativ nachzuweisen oder qualitativ zu bestimmen, werden bevorzugt immunchemische Verfahren angewandt. Dazu werden üblicherweise Antikörper verwendet, die den Analyten spezifisch erkennen. Um beispielsweise ein Enzym nachzuweisen, werden markierte Substrate eingesetzt, die spezifisch von dem nachzuweisenden Enzym modifiziert werden. Um besonders aussagekräftige und genaue Ergebnisse zu erhalten, ist es häufig erforderlich, eine Nachweisreaktion über einen Zeitraum zu beobachten und ihren Verlauf zu analysieren. Die Veränderung einer reaktionsabhängigen Messgröße über die Zeit wird auch als Reaktionskinetik oder Signal-Zeit-Kurve bezeichnet. Verschiedene Parameter einer solchen Kurve können zur Auswertung und damit zur Bestimmung der Konzentration oder Aktivität eines Analyten verwendet werden.

Beispiele für Nachweisreaktionen, deren Reaktionskinetik ausgewertet wird, sind z. B. partikelverstärkte Agglutinationsassays, bei denen partikelgebundene bi- oder polyvalente Antikörper mit einem Antigen reagieren, wodurch es zur Bildung von stark lichtstreuenden Molekülaggregaten kommt. Die analytabhängige Bildung dieser Molekülaggregate kann über die Messung der Streulichtintensität (Nephelometrie) oder über die Messung der Transmissionszunahme oderabnahme (Turbidimetrie) gemessen werden und zur Bestimmung der Konzentration des Analyten genutzt werden. Weitere Beispiele sind Enzymbasierte Testverfahren, bei denen die Umsetzung eines chromogenen Substrats über die Zeit gemessen wird. Die analytabhängige Spaltung eines chromogenen Substrats kann z. B. über die Messung der Transmissionszunahme oder - abnahme bei einer bestimmten Wellenlänge gemessen werden und zur Bestimmung der Konzentration oder Aktivität des Analyten genutzt werden. Ein anderes Beispiel sind Gerinnungsteste, bei denen die Aktivität eines einzelnen oder mehrerer Blutgerinnungsfaktoren durch die Messung der Fibrinbildungsgeschwindigkeit in einer Blut- oder Plasmaprobe bestimmt wird. Typische Beispiele für derartige Gerinnungsteste sind die Prothrombinzeit (PT), die auch Quick-Test oder Thromboplastinzeit genannt wird, die aktivierte partielle Thromboplastinzeit (APTT), die Thrombinzeit (TT), die Batroxobinzeit (BT) oder die Ecarinzeit (ECT).

Verschiedene Parameter einer Reaktionskinetik (Signal-Zeit-Kurve) können für eine Auswertung verwendet werden. Bekannte Parameter sind z. B. die maximale Reaktionsgeschwindigkeit bzw. der Zeitpunkt des Auftretens der maximalen Reaktionsgeschwindigkeit. Häufig wird zur Auswertung einer Reaktionskinetik auch zunächst die erste oder zweite Ableitung der Kinetik gebildet. Ein weiterer bekannter Parameter einer Reaktionskinetik ist z. B. auch die Fläche unter der ersten Ableitung einer Signal-Zeit-Kurve. In anderen Fällen wird der Zeitpunkt bestimmt, bei dem das Signal einen vorbestimmten Schwellenwert überschreitet, oder es wird die Signalhöhe bestimmt, die zu einem vorbestimmten Zeitpunkt erreicht wird. Der übrige Verlauf der Reaktionskurve wird dann in erster Linie zur Überprüfung der Plausibilität der Messwerte ausgewertet.

Im einfachsten Fall eines Verfahrens zur Bestimmung eines Analyten wird eine Probe, die den Analyten enthält, mit einem Reagenz vermischt, das die zum Nachweis des Analyten notwendigen Komponenten, wie z. B. ein Substrat, einen Bindungspartner, einen Aktivator oder ähnliches enthält. Üblicherweise wird die Reaktion ab Zugabe des Reagenzes, d. h. ab dem Zeitpunkt t₀ anhand der Messung eines geeigneten physikalischen Signals verfolgt. Idealerweise verhält sich das Messsignal zu jedem Zeitpunkt proportional zur Reaktion. Tatsächlich kommt es jedoch zu verschiedenen Phänomenen, wie z. B. Ungenauigkeiten des Messinstruments, die dazu führen, dass sich nicht jedes absolute Messsignal proportional zur Reaktion verhält. Üblicherweise werden deshalb z. B. verschiedene Glättungsverfahren verwendet, um eine glattere Reaktionskinetik zu erhalten.

Ein anderes Problem bei der Auswertung von Reaktionskinetiken und somit bei der Bestimmung der Konzentration oder Aktivität eines Analyten besteht darin, dass es unmittelbar nach Zugabe eines Reagenzes zur Probe häufig zunächst zu großen Schwankungen und Unregelmäßigkeiten im Reaktionsverlauf und anschließend zu einem verzögerten Reaktionsbeginn kommt, bevor eine exponentielle Reaktionsphase beginnt, die durch einen relativ steil und stetig ansteigenden Kurvenverlauf, also eine hohe Reaktionsgeschwindigkeit gekennzeichnet ist und die im Allgemeinen den analytisch wichtigsten Reaktionsabschnitt darstellt. Die erste Reaktionsphase hingegen, die auch als Reaktions-Lag-Phase bezeichnet wird, ist gekennzeichnet durch einen flachen Kurvenverlauf, also eine niedrige Reaktionsgeschwindigkeit, und eignet sich gewöhnlich nicht zur Auswertung.

Im Stand der Technik wird in vielen Auswerteverfahren eine festgelegte Zeitspanne t₀ bis tₙ, die die Reaktions-Lag-Phase sicher umfassen soll, von vornherein von der Auswertung ausgeschlossen. Üblicherweise wird die Dauer dieser Zeitspanne durch empirische Untersuchungen für ein spezifisches Testverfahren bestimmt. Dazu wird eine hinreichende Anzahl von Messungen unter den erforderlichen Testbedingungen durchgeführt. In der Praxis hat sich jedoch gezeigt, dass selbst unter gleichbleibenden Testbedingungen die Dauer der Reaktions-Lag-Phase variabel ist. Der Nachteil der Verwendung einer festgelegten Zeitspanne t₀ bis tₙ, die die Reaktions-Lag-Phase sicher umfassen soll, besteht darin, dass in Fällen, in denen die Reaktions-Lag-Phase tatsächlich kürzer ist, Teile der Reaktionskinetik, die sich zur Auswertung eignen würden, nicht verwendet werden können, während in Fällen, in denen die Reaktions-Lag-Phase tatsächlich länger ist, Teile der Reaktionskinetik, die sich zur Auswertung nicht eignen, verwendet werden. In beiden Fällen besteht die Gefahr von Ungenauigkeiten bei der Auswertung der Reaktionskinetik und damit die Gefahr einer fehlerhaften Bestimmung des Analyten.

Der vorliegenden Erfindung lag also die Aufgabe zugrunde ein Verfahren zur Bestimmung der Konzentration oder Aktivität eines Analyten bereit zu stellen, das die spezifische Bestimmung der Reaktions-Lag-Phase einer Reaktionskinetik ermöglicht und damit eine genauere Auswertung der Reaktionskinetik und damit eine genauere Bestimmung eines Analyten gewährleistet.

Die Aufgabe wird erfindungsgemäß durch ein Verfahren gemäß Anspruch 1 gelöst.

Gegenstand der vorliegenden Erfindung ist ein Verfahren zur Bestimmung der Konzentration oder Aktivität eines Analyten in einer Probe, welches folgende Schritte umfasst:
a) Vermischen der Probe mit mindestens einem Reagenz, wodurch eine analytabhängige Reaktion in Gang gesetzt wird;
b) Messung eines sich infolge der analytabhängigen Reaktion ändernden Signals (x) über die Zeit (t) und Speicherung der Signal-Zeit-Kurve;
c) Bestimmung der maximalen Steigung der Signal-Zeit-Kurve;
d) Bestimmung der Reaktions-Lag-Phase der Signal-Zeit-Kurve;
e) Bestimmung der Konzentration oder Aktivität des Analyten anhand mindestens eines Parameters der Signal-Zeit-Kurve, der zeitlich nach der Reaktions-Lag-Phase liegt,
wobei die Bestimmung der Reaktions-Lag-Phase erfolgt, indem die Zeitspanne vom Zeitpunkt des ersten Signals (t₀) bis zum Zeitpunkt t_{lag} bestimmt wird. Dazu wird zunächst der Zeitpunkt tₘₐₓ der maximalen Steigung der Signal-Zeit-Kurve bestimmt. Anschließend wird - ausgehend vom Zeitpunkt tₘₐₓ- der Zeitpunkt t_{lag} bestimmt, wobei t_{lag} kleiner ist als tₘₐₓ, d. h. zeitlich vor tₘₐₓ liegt und wobei t_{lag} dem Zeitpunkt entspricht, an dem die Steigung der Signal-Zeit-Kurve - ausgehend von tₘₐₓ - einen Grenzwert erstmalig unterschreitet. Der Grenzwert entspricht einem testspezifischen, vorbestimmten Bruchteil von der maximalen Steigung der Signal-Zeit-Kurve. Bevorzugterweise entspricht der Grenzwert einem Anteil von 1 bis 45 % der maximalen Steigung der Signal-Zeit-Kurve.

Die Messung des sich infolge der analytabhängigen Reaktion ändernden Signals (x) über die Zeit (t) erfolgt mit Hilfe geeigneter Detektionsvorrichtungen. Bei dem Signal kann es sich um jede dem Fachmann bekannte Signalart handeln, wie z. B. um optische Eigenschaften des Reaktionsansatzes (z. B. Trübung, Lichtstreuung, Fluoreszenz, Chemilumineszenz), um chemische Eigenschaften des Reaktionsansatzes (z. B. pH-Wert, Redoxpotenzial, Viskosität, Festigkeit) oder um elektrochemische Eigenschaften (z. B. Leitfähigkeit, Impedanz, Widerstand).

Die Messung über die Zeit (t) erfolgt üblicherweise durch die kontinuierliche Erfassung des physikalischen Signals, bevorzugt in gleichmäßigen zeitlichen Abständen, wie z. B. eine Messung pro Sekunde. Die so erhaltene Signal-Zeit-Kurve wird gespeichert, um der nachfolgenden Auswertung zur Verfügung zu stehen. Der Begriff "Signal-Zeit-Kurve" ist in diesem Zusammenhang breit zu verstehen und nicht auf die graphische Darstellung einer entsprechenden Kurve beschränkt. Vielmehr ist damit die Zuordnung der Messwerte zu den dazugehörigen Zeitpunkten in einer geordneten Reihe gemeint.

Die Bestimmung der maximalen Steigung der Signal-Zeit-Kurve kann durch jedes beliebige dem Fachmann bekannte Verfahren erfolgen, bevorzugterweise jedoch, indem zunächst die 1. Ableitung der Signal-Zeit-Kurve gebildet wird und das Maximum der 1. Ableitung bestimmt wird. Der Zeitpunkt des Maximums der 1. Ableitung entspricht dem Zeitpunkt tₘₐₓ der maximalen Steigung der Signal-Zeit-Kurve.

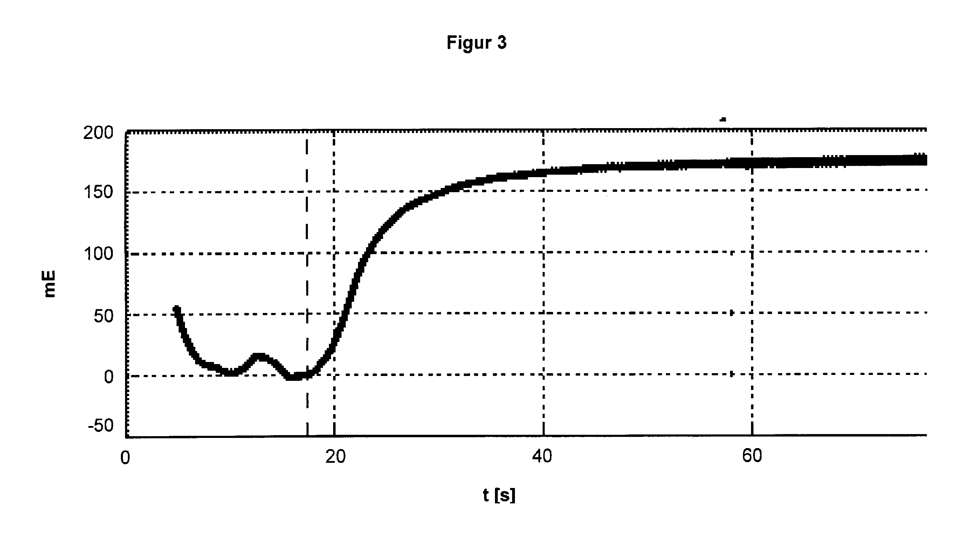

Die Bestimmung des Zeitpunkts t_{lag}, der das Ende der Reaktions-Lag-Phase festlegt, erfolgt, indem ausgehend von tₘₐₓ in der Signal-Zeit-Kurve nach dem Zeitpunkt gesucht wird, an dem die Steigung der Signal-Zeit-Kurve einen Grenzwert erstmalig unterschreitet, wobei dieser Zeitpunkt kleiner sein muss als tₘₐₓ, d. h. zeitlich vor tₘₐₓ liegen muss. Die Tatsache, dass ausgehend von tₘₐₓ nach dem Zeitpunkt gesucht wird, an dem die Steigung der Signal-Zeit-Kurve einen Grenzwert erstmalig unterschreitet, hat den Vorteil, dass eventuelle Extremschwankungen ganz zu Beginn der Messwertaufnahme nicht zu einer falschen Bestimmung der Reaktions-Lag-Phase führen können. Würde man ausgehend von t₀ nach dem Zeitpunkt suchen, an dem die Steigung der Signal-Zeit-Kurve einen Grenzwert erstmalig überschreitet, könnte eine solche Extremschwankung, die gelegentlich ganz zu Beginn der Messung aufgrund von Vermischungsvorgängen zwischen Probe und Reagenz auftreten kann, zur Bestimmung einer falsch zu kurzen Reaktions-Lag-Phase führen (siehe z. B. Fig. 3).

Der Grenzwert entspricht einem testspezifischen, vorbestimmten Bruchteil von der maximalen Steigung der Signal-Zeit-Kurve. Welcher Bruchteil der maximalen Steigung der Signal-Zeit-Kurve sich für ein bestimmtes Testverfahren eignet, ist durch eine einfache Versuchsreihe vorab empirisch zu bestimmen. Stimmen die mit Hilfe eines vorläufigen Grenzwertes berechnete Reaktions-Lag-Phase und die erwartete Reaktions-Lag-Phase überein und lässt sich diese Übereinstimmung in einer Vielzahl von Probemessungen bestätigen, ist ein geeigneter Grenzwert gefunden. Bevorzugterweise wird der Grenzwert so gewählt, dass er einem Anteil von 1 bis 45 % der maximalen Steigung der Signal-Zeit-Kurve entspricht. Allgemein kann man sagen, dass für den Grenzwert ein kleinerer Bruchteil der maximalen Steigung der Signal-Zeit-Kurve zu wählen ist, je steiler die Signal-Zeit-Kurve bei einem bestimmten Testverfahren üblicherweise ansteigt.

Nachdem Beginn und Ende der Reaktions-Lag-Phase (t₀ bis t_{lag}) bestimmt wurden, erfolgt die Bestimmung der Konzentration oder Aktivität des Analyten anhand mindestens eines Parameters der Signal-Zeit-Kurve, der zeitlich nach der Reaktions-Lag-Phase liegt.

Unter einer "Probe" ist im Sinne der Erfindung das Material zu verstehen, das die nachzuweisende Substanz, d. h. den Analyten vermutlich enthält. Der Begriff "Probe" umfasst beispielsweise biologische Flüssigkeiten oder Gewebe insbesondere von Menschen und Tieren wie Blut, Plasma, Serum, Speichel, Sputum, Exudat, bronchoalveoläre Lavage, Lymphflüssigkeit, Synovialflüssigkeit, Samenflüssigkeit, Vaginalschleim, Faeces, Urin, Liquor, Haare, Haut, Gewebeproben oder -schnitte. Ferner sind umfasst Zellkulturproben, pflanzliche Flüssigkeiten oder Gewebe, forensische Proben, Wasser- und Abwasserproben, Nahrungsmittel, Arzneimittel. Gegebenenfalls müssen die Proben vorbehandelt werden, um den Analyten für das Nachweisverfahren zugänglich zu machen oder um störende Probenbestandteile zu entfernen. Solche Vorbehandlung von Proben mag die Abtrennung und/oder Lyse von Zellen beinhalten, das Präzipitieren, die Hydrolyse oder die Denaturierung von Probenbestandteilen wie z. B. Proteinen, die Zentrifugation von Proben, das Behandeln der Probe mit organischen Lösungsmitteln wie z. B. Alkohole, insbesondere Methanol; das Behandeln der Probe mit Detergenzien. Häufig wird die Probe in ein anderes, meistens wässriges Medium überführt, welches mit dem Nachweisverfahren möglichst nicht interferieren soll.

Unter dem Begriff "Analyt" ist im Sinne der Erfindung die nachzuweisende Substanz zu verstehen (Beispiele für den Begriff "Analyt" siehe EP 515 194 A2, Seiten 8-15) oder auch ein biologischer Parameter, der Aufschluss über den Zustand eines physiologischen Prozesses bzw. eines mehrstufigen, multifaktoriellen Reaktionssystems gibt. Beispiele für derartige biologische Parameter sind z. B. die verschiedenen bekannten Gerinnungszeiten, wie z. B. die Prothrombinzeit (PT) oder die aktivierte partielle Thromboplastinzeit (APTT), die Informationen über die verschiedenen Teilbereiche der Blutgerinnungskaskade oder des Fibrinolysesystems liefern.

In bevorzugten Ausführungsformen des erfindungsgemäßen Verfahrens handelt es sich bei der analytabhängigen Reaktion um eine Antigen-Antikörper-Reaktion oder eine Enzym-Substrat-Reaktion.

Weiterhin bevorzugt sind Verfahren, bei denen die analytabhängige Reaktion die Bildung eines Fibringerinnsels in einer Vollblut- oder Plasmaprobe ist.

Besonders bevorzugt ist ein erfindungsgemäßes Verfahren zur Bestimmung der Konzentration oder Aktivität von Thrombin, bevorzugt von endogenem Thrombin. Dazu wird die Probe üblicherweise mit einem Reagenz zur Aktivierung von Prothrombin zu Thrombin vermischt. Um die Thrombinbildung zu induzieren, können z. B. Lösungen verwendet werden, die Ca²⁺-lonen und zusätzlich z. B. Thromboplastin oder einen Kontaktaktivator, wie z. B. Kaolin, Phospholipide, Schlangengift, oder Thrombomodulin und aktiviertes Protein C enthalten. Je nach diagnostischer Fragestellung kann der Fachmann aus der Vielzahl bekannter Aktivatoren der Blutgerinnung wählen, um entweder einen Teilbereich oder das gesamte Gerinnungssystem zu betrachten. Die zur Bestimmung des endogenen Thrombins notwendige Messung der Umsatzkinetik eines Thrombinsubstrats erfordert, dass die zu untersuchende Probe mit einem Thrombinsubstrat versetzt wird, dass die Thrombinbildung induziert wird, z. B. durch Zugabe eines Thrombinbildungsaktivators, und dass eine physikalische oder chemische Eigenschaft des umgesetzten Thrombinsubstrats als Funktion der Zeit gemessen wird (siehe auch EP 420 332 B1).

Als Thrombinsubstrate eignen sich z. B. Oligopeptide, die sich aus einem Teil, der eine spezifische Erkennungssequenz für Thrombin umfasst, und aus einer abgehenden Signalgruppe mit einer messbaren physikalischen Eigenschaft zusammensetzen. Bei der abgehenden Signalgruppe, die bevorzugterweise nach Abspaltung über eine veränderte physikalische Eigenschaft verfügt, kann es sich z. B. um eine chromophore, chemilumineszierende oder fluoreszente Gruppe handeln, deren Eigenschaft gemessen werden kann. Bevorzugt sind chromophore Signalgruppen deren optische Eigenschaft photometrisch bestimmt wird, wie z. B. para-Nitroanilid (pNA), dessen Absorption nach Abspaltung durch Thrombin bei einer Wellenlänge von 405 nm gemessen werden kann.

Bevorzugterweise erfolgt die Bestimmung der Konzentration oder Aktivität von endogenem Thrombin anhand des sogenannten endogenen Thrombinpotenzials (ETP). Dazu wird, nachdem die Reaktions-Lag-Phase bestimmt wurde, die Fläche unter der ersten Ableitung der Signal-Zeit-Kurve bestimmt. Eine besonders bevorzugte Variante der Bestimmung des endogenen Thrombinpotenzials ist in EP 1 669 761 A2 beschrieben.

Ein weiterer Gegenstand der Erfindung ist ein Gerät, das imstande ist, das erfindungsgemäße Verfahren zur Bestimmung der Aktivität oder Konzentration eines Analyten automatisch durchzuführen. Eine solches Gerät zeichnet sich dadurch aus, dass es a) über Mittel zur Messung des sich infolge der analytabhängigen Reaktion ändernden Signals (x) über die Zeit (t) verfügt (z. B. ein Photometer, pH-Meter, Dosimeter, Luminometer, Fluorimeter oder ähnliches), über b) Mittel zur Speicherung der Signal-Zeit-Kurve (z. B. einen Halbleiterspeicher, ein optisches oder magnetisches Speichermedium wie bspw. eine Festplatte), über c) Mittel zur Bestimmung des Zeitpunkts tₘₐₓ der maximalen Steigung der gespeicherten Signal-Zeit-Kurve (z. B. Software, Computerprogramm, Algorithmus) sowie über Mittel zur Steuerung der Durchführung des Verfahrensschritts ii) des Verfahrens gemäß Anspruch 1 zur Bestimmung des Zeitpunkts t_{lag} (z. B. Software, Computerprogramm, Algorithmus). Bevorzugterweise verfügt das Gerät zusätzlich über Mittel zur Ausgabe von Messergebnissen (z. B. ein elektronisches Anzeigegerät, einen Monitor, einen Datenschreiber, einen Drucker und/oder Datenfernleitung).

### Figurenbeschreibung

### Figur 1

Bestimmung der Reaktions-Lag-Phase einer Signal-Zeit-Kurve einer Thrombingenerierungsreaktion in einer Plasmaprobe, die durch Zugabe von Innovin® gestartet wurde. Das obere Diagramm zeigt die Messwerte [mE] über die Zeit. Die gestrichelte vertikale Linie markiert den Zeitpunkt t_{lag}. Das untere Diagramm zeigt die 1. Ableitung dieser Reaktionskinetik. Die gestrichelte vertikale Linie markiert den Zeitpunkt des Maximums der 1. Ableitung, welcher dem Zeitpunkt tₘₐₓ der maximalen Steigung der Signal-Zeit-Kurve entspricht. Das Maximum der 1. Ableitung ist cₘₐₓ = 102,86 mE/min. Für den Grenzwert waren 20 % des maximalen Signals der 1. Ableitung vorab bestimmt worden. Der sich daraus ergebende Grenzwert von 20,572 mE/min (durchgezogene horizontale Linie) wird ausgehend von tₘₐₓ erstmalig bei 0,503 min (= 30,2 s) unterschritten. Dieser Zeitpunkt entspricht t_{lag}.

### Figur 2

Bestimmung der Reaktions-Lag-Phase einer Signal-Zeit-Kurve einer Thrombingenerierungsreaktion in einer Plasmaprobe, die durch Zugabe von Actin@ FS gestartet wurde. Die gestrichelte vertikale Linie markiert den Zeitpunkt t_{lag} bei 960,2 Sekunden.

### Figur 3

Bestimmung der Reaktions-Lag-Phase einer Signal-Zeit-Kurve einer Prothrombinzeit-Gerinnungsreaktion in einer Plasmaprobe, die durch Zugabe von Innovin® gestartet wurde. Die gestrichelte vertikale Linie markiert den Zeitpunkt t_{lag} bei 17,3 Sekunden.

Die im Folgenden beschriebenen Beispiele dienen der exemplarischen Beleuchtung einzelner Aspekte dieser Erfindung und sind nicht als Einschränkung zu verstehen:

### Beispiele

Auf einem automatischen Gerinnungsanalyzer (BCS® System, Dade Behring Marburg GmbH, Marburg, Deutschland) wurden verschiedene bekannte Testverfahren zur Bestimmung verschiedener Analyten in humanen Plasmaproben durchgeführt. Auf dem Gerät wurde zusätzlich eine Software installiert, die die automatische Durchführung des erfindungsgemäßen Verfahrens zur Bestimmung der Reaktions-Lag-Phase ermöglichte.

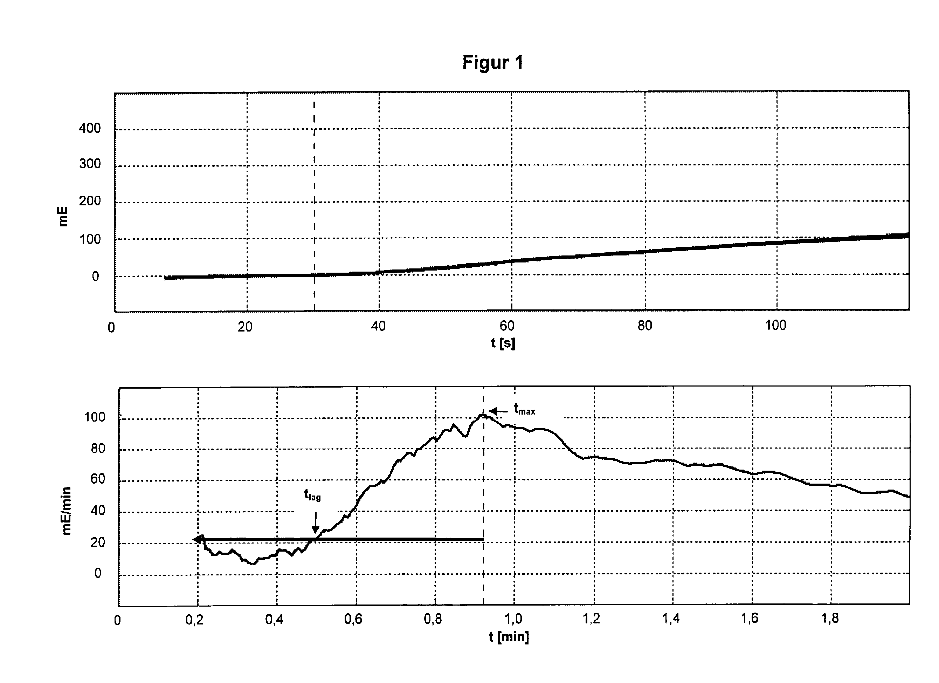

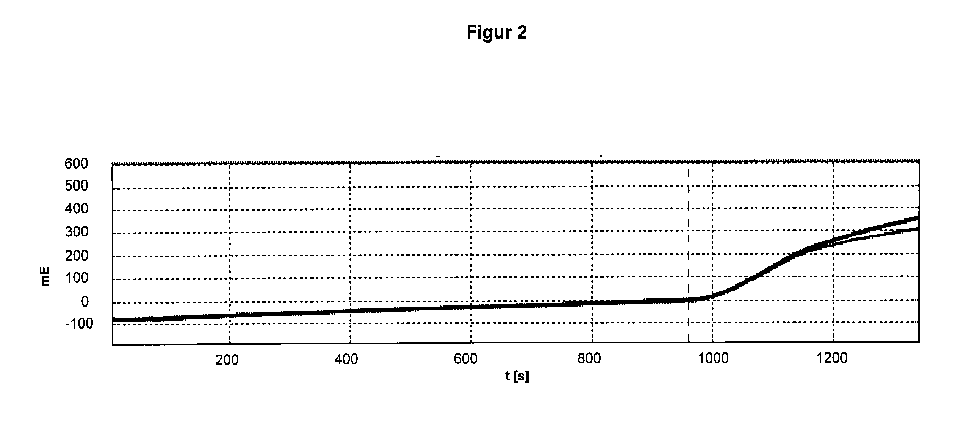

Zur Bestimmung der Thrombingenerierung wurde eine Plasmaprobe mit einem para-Nitroanilid-gekoppelten thrombinspezifischen Peptidsubstrat vermischt, und die Thrombingenerierung wurde durch Zugabe von Innovin® (Reagenz bestehend aus rekombinantem, humanem Gewebefaktor und einer Mischung von synthetischen Phospholipiden; Dade Behring Marburg GmbH, Marburg, Deutschland) und CaCl₂ oder durch Zugabe von Actin@ FS (APTT-Reagenz, Dade Behring Marburg GmbH, Marburg, Deutschland) und CaCl₂ gestartet. Die Reaktionskinetiken sind in Figur 1 bzw. Figur 2 dargestellt. Die Figur 1 zeigt hierbei den vergrößerten Auschnitt der ersten 100 Sekunden Messzeit. Die Gesamtmesszeiten betrugen im Falle der Aktivierung mit Innovin® 20 Minuten und im Falle der Aktivierung mit Actin@ FS 50 Minuten. Die Lag-Phase wurde bei Verwendung von Innovin® auf 30,2 Sekunden (Fig. 1) und bei Verwendung von Actin@ FS auf 960,2 Sekunden (Fig. 2) bestimmt.

Zur Bestimmung der Prothrombinzeit (PT) wurde eine Plasmaprobe mit Innovin® gemäß Herstellerangaben vermischt. Die Gesamtmesszeit betrug 120 Sekunden. Die Lag-Phase wurde auf 17,3 Sekunden bestimmt. Das erfindungsgemäße Verfahren zur Bestimmung der Reaktions-Lag-Phase verhält sich robust gegenüber Signalstörungen und Signalrauschen am Beginn der Messung und wertet die Lag-Phase korrekt aus (Fig. 3).

## Patentansprüche

1. Verfahren zur Bestimmung der Konzentration oder Aktivität eines Analyten in einer Probe, das folgende Schritte umfasst:
a) Vermischen der Probe mit mindestens einem Reagenz, wodurch eine analytabhängige Reaktion in Gang gesetzt wird;
b) Messung eines sich infolge der analytabhängigen Reaktion ändernden Signals (x) über die Zeit (t) und Speicherung der Signal-Zeit-Kurve;
c) Bestimmung der maximalen Steigung der Signal-Zeit-Kurve;
d) Bestimmung der Reaktions-Lag-Phase der Signal-Zeit-Kurve;
e) Bestimmung der Konzentration oder Aktivität des Analyten anhand mindestens eines Parameters der Signal-Zeit-Kurve, der zeitlich nach der Reaktions-Lag-Phase liegt,
**dadurch gekennzeichnet, dass**
die Bestimmung der Reaktions-Lag-Phase erfolgt, indem die Zeitspanne vom Zeitpunkt des ersten Signals (t₀) bis zum Zeitpunkt t_{lag} bestimmt wird, wobei
i) zunächst der Zeitpunkt tₘₐₓ der maximalen Steigung der Signal-Zeit-Kurve bestimmt wird;
ii) dann ausgehend vom Zeitpunkt tₘₐₓ der Zeitpunkt t_{lag} bestimmt wird, wobei t_{lag} kleiner ist als tₘₐₓ und wobei t_{lag} dem Zeitpunkt entspricht, an dem die Steigung der Signal-Zeit-Kurve einen Grenzwert erstmalig unterschreitet und wobei der Grenzwert einem testspezifischen, vorbestimmten Bruchteil von der maximalen Steigung der Signal-Zeit-Kurve entspricht.

2. Verfahren gemäß Anspruch 1 **dadurch gekennzeichnet, dass** der Grenzwert einem Bruchteil von 1 bis 45 % der maximalen Steigung der Signal-Zeit-Kurve entspricht.

3. Verfahren gemäß einem der vorhergehenden Ansprüche, wobei die Konzentration oder Aktivität eines Analyten in einer biologischen Probe aus der Gruppe Vollblut, Plasma, Serum, Urin, Faeces, Speichel oder Liquor bestimmt wird.

4. Verfahren gemäß einem der vorhergehenden Ansprüche, wobei die analytabhängige Reaktion eine Antigen-Antikörper-Reaktion oder eine Enzym-Substrat-Reaktion ist.

5. Verfahren gemäß einem der vorhergehenden Ansprüche, wobei ein Signal aus der Gruppe Streulicht, Trübung, Fluoreszenz und Chemilumineszenz gemessen wird.

6. Verfahren gemäß einem der vorhergehenden Ansprüche zur Bestimmung der Konzentration oder Aktivität von Thrombin.

7. Verfahren gemäß Anspruch 6, wobei die Probe mit einem Reagenz zur Aktivierung von Prothrombin zu Thrombin vermischt wird.

8. Verfahren gemäß einem der Ansprüche 6 und 7, wobei die Probe mit einem Reagenz enthaltend ein Thrombinsubstrat vermischt wird.

9. Verfahren gemäß Anspruch 8, wobei ein sich infolge der thrombinabhängigen Substratspaltung änderndes Signal (x) über die Zeit (t) gemessen wird und die Signal-Zeit-Kurve gespeichert wird.

10. Verfahren gemäß einem der Ansprüche 6 bis 9 zur Bestimmung der Bildung und Inhibierung von endogenem Thrombin.

11. Verfahren gemäß Anspruch 1 zur Bestimmung einer Gerinnungszeit.

12. Verfahren gemäß Anspruch 11 zur Bestimmung einer Gerinnungszeit aus der Gruppe Prothrombinzeit, aktivierte partielle Thromboplastinzeit, Thrombinzeit, Batroxobinzeit und Ecarinzeit.

13. Gerät zur automatischen Durchführung eines Verfahrens zur Bestimmung der Konzentration oder Aktivität eines Analyten in einer Probe enthaltend
a) Mittel zur Messung des sich infolge der analytabhängigen Reaktion ändernden Signals (x) über die Zeit (t),
b) Mittel zur Speicherung der Signal-Zeit-Kurve, sowie
c) Mittel zur Bestimmung des Zeitpunkts tₘₐₓ der maximalen Steigung der gespeicherten Signal-Zeit-Kurve
**dadurch gekennzeichnet, dass** es zusätzlich ein Mittel zur Steuerung der Durchführung des Verfahrensschritts ii) des Verfahrens gemäß Anspruch 1 zur Bestimmung des Zeitpunkts t_{lag} enthält.

14. Gerät gemäß Anspruch 13 **dadurch gekennzeichnet, dass** das Mittel zur Steuerung der Durchführung des Verfahrensschritts ii) des Verfahrens gemäß Anspruch 1 eine installierte Software ist.
